## Europäisches Patentamt

## European Patent Office

## Office européen des brevets

(11) Publication number: **0 002 090**
**A1**

(12) **EUROPEAN PATENT APPLICATION**

(21) Application number: **78200301.6**

(51) Int. Cl.²: **A 61 L 17/00**

(22) Date of filing: **13.11.78**

---

(30) Priority: **22.11.77 US 853848**

(43) Date of publication of application: **30.05.79**
**Bulletin 79/11**

(84) Designated Contracting States: **CH DE FR GB**

(71) Applicant: **GENERAL ATOMIC COMPANY, 10955 John Jay Hopkins Drive, San Diego California, 92121 (US)**

(72) Inventor: **Bokros, Jack Chester, 2262 Peutz Valley Road, Alpine California 92001 (US)**
Inventor: **Shim, Hong Seop, 10978 Janice Court, San Diego California 92126 (US)**
Inventor: **Haubold, Axel Dietrich, 3462 Aldford Drive, San Diego California 92111 (US)**

(74) Representative: **Keuzenkamp, Abraham et al, P.O. Box 302, NL-2501 CH Den Haag (NL)**

---

(54) **Multifibre suture.**

(57) A multifibre carbon coated suture comprises a plurality of small diameter fibres having a tensile modulus of at least about $13.8 \times 10^6$ kPa and a thin, smooth, adherent, isotropic carbon coating on the substrate fibre having particular properties including a tensile fracture strain of at least about 5%.

1

MULTIFIBRE SUTURE

This invention relates to multifibre sutures adapted for prolonged or permanent implantation in a living body, and, more particularly is directed to multistrand sutures having a carbon coating which may be utilized in surgical procedures.

The employment of pyrolytic carbon coatings to produce biocompatible and thromboresistant surfaces has produced substantial advancement in the medical field and is described, for example, in U.S. Patent Specifications 3,526,005 and 3,685,059. These patent specifications generally describe deposition of pyrolytic carbon coatings, usually from a diluted hydrocarbon atmosphere at atmospheric pressure. Various other techniques have been developed for depositing vapour coatings, for example, as by vacuum vapour deposition (VVD) which is also sometimes referred to as vacuum metallizing, physical vapour deposition or evaporative coating, sputtering or as by ion-plating techniques (e.g., see Marinkovic, et al., Carbon, 14, 329, (1976)). Coatings deposited by such techniques, which are generally referred to herein as vapour-deposited carbon coatings, have been utilized in prosthetic devices, as described in U.S. Patent Specification 3,952,334. However, there is still a need to provide other elements used in the medical field, such as sutures with biocompatibility.

Conventionally, sutures used in many surgical procedures are made of material which is capable of being absorbed by the body over a period of time. However, a number of surgical procedures utilize, or could benefit from, sutures which are intended to be permanently retained in the body to provide a supportive or structural function. For example, sutures may be used to connect a prosthetic heart valve to heart tissue, or to connect synthetic or biologic grafts into the vascular system.

Accordingly, it is an object of the invention to provide sutures which are suitable for prolonged or permanent implantation in a living body and which are capable of providing substantial structural strength.

In accordance with the invention, a multifibre suture for prolonged or permanent implantation in a living body comprises a fibre substrate array of a plurality of organo-polymeric fibres having a tensile modulus of elasticity of at least $13.8 \times 10^{6}$ kPa, and a fibre diameter of less than 25 µm, and a dense, isotropic carbon coating on the fibres having a BAF (Bacon Anisotropy Factor) of not more than 1.3, a thickness of less than 0.7 µm, and a tensile fracture strain of at least 5 per cent. This improved suture may suitably be used for reconstructive surgery. The multifibre sutures may desirably be provided in braided or helically wound, stranded form, and may be provided with a range of suture diameters for different surgical applications.

The flexible multifibre sutures comprise a plurality of intertwined carbon-coated, organopolymeric fibres of particular characteristics. Generally, the sutures will comprise at least about 7 such fibres. The organopolymeric fibres are of relatively small diameter which are able to sustain the functional stresses intended for the particular suture utilization and provide for a desired high degree of tensile strength and flexibility without straining more than about 5 per cent. The individual fibres of the multifibre suture should generally best have a major diameter dimension of less

than about 25 μm, and a minor diameter dimension of at least about 5 μm, although fibres as small as 1 μm might be used in certain applications. By "major diameter dimension" and "minor diameter dimension" is meant the greatest respectively the smallest dimension of the fibre in a direction orthogonal to the longitudinal axis of the fibre. Of course, for a fibre of circular cross section, the major and minor diameter dimensions will be the same, but it should be appreciated that the invention does contemplate fibres of non-circular cross-section. However, deviation from circular fibre cross-sections generally tends to lead to stiffer sutures because of the increased interfibral friction and increased forces required for bending and unbending of the fibre filaments.

A plurality of individual, thin carbon-coated fibres are combined to provide a flexible strand having a predetermined high degree of flexibility and strength as well as beneficial biologically compatible properties of a carbon coating.

The relatively high degree of flexibility of the composite multifibre suture is due primarily to the bending of the individual fibres with maintenance of the integrity of a carbon coating on the fibres. The radius of curvature of the individual fibres that will provide a degree of bending without fracture of an adherent carbon coating having a relatively high tensile fracture strain of at least 5 per cent, is determined by the diameter of the fibre. The minimum radius of curvature R for a given fibre is approximately represented as follows:

$$R = \frac{\text{diameter of the fibre}}{2 \times \text{allowable strain}}$$

For example, for a fibre diameter of about 10 μm (= $10^{-3}$ cm), the allowable radius of curvature R at an allowable strain of 5% is:

$$R = \frac{1 \times 10^{-3} \text{ cm}}{2 \times (5 \times 10^{-2})} = 0.01 \text{ cm}$$

4

Accordingly, the relatively small fibre diameters of the fibres utilized, provides the composite sutures with substantial flexibility range without cracking the specified carbon coating (which is provided in an isotropic form capable of withstanding at least about 5% strain without fracture). Thinner fibres are preferred for increased flexibility, and the lower limit of diameter is determined by handling and coating parameters.

Certain physical parameters characterize the organo-polymeric substrate fibres of the sutures. In this connection, the fibres should be of an organopolymeric material having a tensile strength of at least about $13.8 \times 10^4$ kPa and should be fabricated of medical grade materials. Generally, the fibres will best have a high degree of axial orientation. The modulus is an important parameter, and the organopolymeric fibres should have a tensile modulus of elasticity of about $13.8 \times 10^6$ kPa or more. Polyethylene terephthalate fibres, such as those sold under the trade name Dacron, are particularly preferred because of the biocompatibility of such polyester fibres ("Implants in Surgery", D. Williams, et al., W.B. Saunders Company Ltd., London, 1973), their strength (e.g., $34.5 \times 10^4$ to $68.3 \times 10^4$ kPa breaking strength) and stiffness (e.g., modulus of elasticity of about $13.8 \times 10^6$ kPa), which may be almost equal to that of the isotropic carbon coating. Such a high modulus, high strength material can support a relatively large load without straining more than 5% (such that the carbon coating would break). Polyethylene terephthalate fibres may be, for example, about three times tougher and five times stiffer than poly(tetrafluorethylene).

As indicated, the sutures comprise a plurality of (generally coaxial) individual, small diameter carbon-coated fibres. Usually such multifibre sutures will comprise at least 7 and preferably at least 15 individual fibres, with the individual fibres desirably being formed into a braided or helically wound or corded form.

In suturing applications where it is desirable to have permanent tissue affixation to the suture and correspondingly limited suture mobility, sutures comprising a braided fibre array may be utilized. However, where because of tissue ingrowth suture immobility is not particularly appropriate, sutures comprising a twisted strand of a plurality of individual carbon-coated fibres may be more desirable.

In addition to poly(ethylene terephthalate), other suitable high strength, high modulus organopolymeric substrate materials, provided their biocompatibility is demonstrated, include various so-called "high temperature polymers" which have generally been developed in the last decade, such as the high modulus and high tensile strength aromatic polyimides and aromatic polyamides. High temperature polymer fibres which may be used herein exhibit thermal stability at temperatures of $300^{\circ}C$ and higher and are generally characterized as high temperature, high molecular weight, aromatic, nitrogen-linked polymers. Such polymers are well known in the polymer art, and examples of such high temperature polymers include ordered aromatic copolyamides, such as the reaction product of phenylene-bis-(amino-benzamide) and isophthaloyl chloride, all-aromatic polybenzimidazoles, such as poly[2,2'-(m-phenylene)-5,5'-(6,6'-benzimidazoles)], polyozadiazoles, poly-(n-phenyl triazoles), polybenzobenzimidazoles, polyimides and poly(amide-imide) resins. Of course, the biocompatibility of such materials should be tested, and medical implant grade materials should be used for prosthetic implants. The preferred organopolymeric fibres contemplated for use herein are medical grade polyethylene terephthalates, but various conventional high temperature polymer fibres commercially available, such as fibres sold under the name Kevlar by DuPont, and having a modulus of about $69 \times 10^6$ kPa may prove useful.

The individual fibres of the sutures are provided with an adherent isotropic carbon coating of particular properties including high fracture strain and specified isotropy and thickness.

The carbon coatings may be provided on suitable substrate fibres by vapour-deposition techniques such as described in the previously referenced U.S. Patent Specification 3,952,334, to produce strongly adherent carbon coatings which have outstanding tissue and blood compatibility. The carbon should be provided in a very highly elasticity form having 5 per cent or more fracture strain.

The individual fibres will typically be about 10 μm in diameter. The thinner the fibre, the smaller the radius of curvature it can sustain without cracking the specified carbon coating, which is required to be capable of sustaining at least about 5% elastic strain before fracture, as previously discussed. In view of the small diameter of the fibres used, it is an advantage that the carbon coating may be provided either by coating the fibres before assembly in multifibre suture form, or by coating the suture fibre assembly. However, it will be appreciated that braiding or twisting of previously coated fibres may generally tend to introduce bending strain, while coating of previously braided or twisted fibres produces a minimum of strain in the finished composite structure and therefore is particularly preferred, particularly in braided suture structures.

In any event, the entire suture structure is provided with a carbon layer of particular properties and may be applied while using coating technology, such as described in U.S. Patent Specification 3,952,334. Further, in this connection, the carbon coating should be at least about 0.1 μm thick, should be adherent, and in order to provide for large fracture strains, should have a BAF (Bacon Anisotropy Factor) of about 1.3 or less and preferably about 1.2 or less. Generally, a coating thickness of about 0.1 to 0.7 μm and preferably from about 0.3 to about 0.5 μm of intermediate density of carbon (at

7

least about 1.6 g/cm$^3$) is employed; greater thicknesses tend to crack and flake. Preferably, the vapour-deposited carbon has a density of about 1.8 g/cm$^3$, and the density should not exceed about 2.0 g/cm$^3$. Such vapour-deposited carbon exhibits biocompatible properties and also may be provided with excellent adherence to the small polymer fibres of the high modulus organopolymeric fibre substrate. As a result, the carbon-coated fibres exhibit excellent properties for use in sutures and are considered to be fully acceptable for implantation within the human body in flexible and tensile service.

Through the design provision of a limited tensile strain in the individual organopolymeric substrate fibres of not more than 5% under intended conditions of use, the integrity of the carbon coating is generally preserved for prolonged or permanent implantation service. In this regard, as previously indicated, small oriented polyethylene terephthalate fibres (e.g., medical grade Dacron) having a high stiffness and high strength are preferred. Other polymers such as aromatic polymers (e.g., aromatic polymer of DuPont having a Kevlar tensile modulus of 69 x 10$^6$ kPa) may also be useful in thin fibre form. Thus, multifibre sutures may be provided which have a high degree of flexibility together with long-term biocompatibility and physical integrity.

The invention will be explained further by way of example with particular reference to the accompanying drawings, in which:

Figure 1 is a view of one embodiment of a multifibre suture in accordance with the invention in open heart operative procedure;

Figure 2 is an enlarged view of a portion of the suture illustrated in Figure 1, and

Figure 3 is a view of another embodiment of a suture in accordance with the invention in operative procedure.

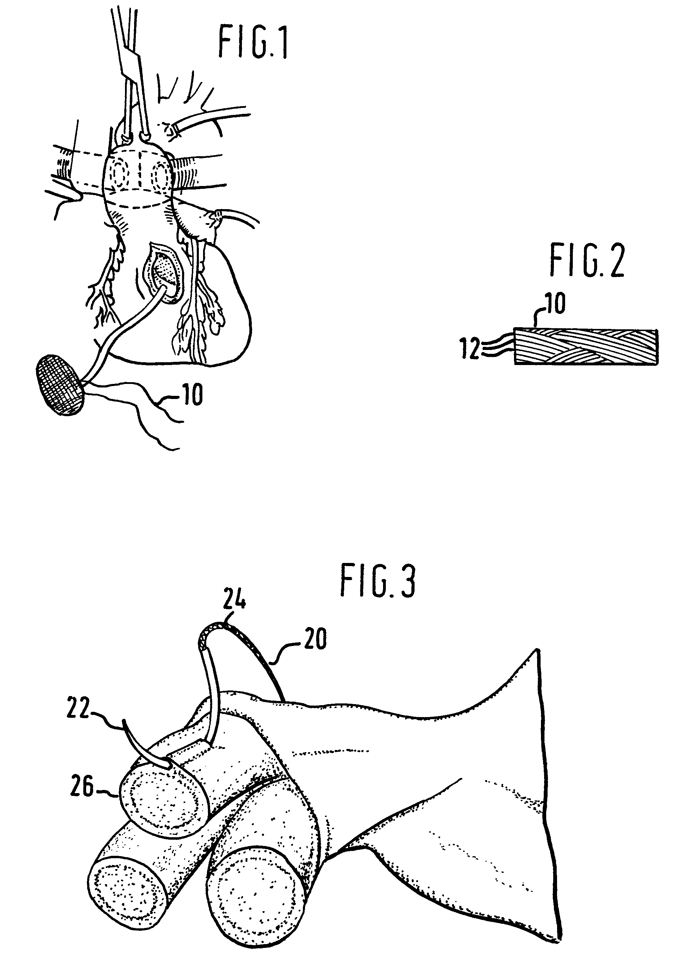

Illustrated in Figure 1 is a step in the operative procedure for correction of truncus arteriosus in which patch closure of the ventricular septal defect is begun by means of a small diameter multistrand suture 10. The suture 10 comprises a braided array of a plurality of small diameter carbon-coated fibres. In the illustrated embodiment, an enlarged view of a portion of suture 10 is shown in Figure 2. The multistrand suture 10 comprises a plurality of 50 individual fibres 12 each having a diameter of about 10 µm. The illustrated multi-strand suture accordingly has a diameter of about 100 µm.

The individual substrate fibres 12 are of circular cross-section and are made of axially oriented polyethylene terephthalate. The individual substrate fibres 12 have a tensile strength of about $27.6 \times 10^{4}$ kPa and a tensile modulus of about $13.8 \times 10^{6}$ kPa.

The substrate fibres 12 have an adherent, carbon surface coating. The coating on the fibres is isotropic carbon having a BAF of about 1.3 or less and a maximum thickness of about 0.3 µm over fibres at the exterior surface of the braided array. The illustrated suture 10 is coated with carbon in finished, braided form, so that the coating thickness on the fibres tends to decrease at the surfaces of the substrate fibres towards the interior of the braided suture 10. Although the fibres of the braided array are coated together in finished form, the fibres are individually coated by the deposition process, and are not substantially bonded together.

In use, the suture 10 is flexible and fatigue-resistant and is biologically compatible in the implantation environment. Further, the braided structure of the graft permits some tissue ingrowth to provide for effective and natural fixation of the permanent suture through immobilization in the sutured tissue. Further, while the carbon coating may be cracked under very high strain areas at suture knots, the carbon coating generally maintains its integrity under conditions of flexure and tensile strain.

9

Illustrated in Figure 3 is an embodiment 20 of a small diameter carbon-coated multifibre suture comprising a braided strand 24 of polyethylene terephthalate fibres, which is shown in operative procedure for perineural suture of lacerated nerve 26 by means of a needle 22. Another example of the use of sutures in accordance with the present invention where permanent retention of a strong, functional suture is appropriate, is the suturing of tendons. A suitable suture for tendon attachment comprises a plurality of 100 small organopolymeric fibres each having a circular cross-section and a diameter of about 5 μm. The fibres may be twisted into a plurality of three substrands which, in turn may be twisted into a multistrand suture array. The preformed multistrand suture substrate is coated in a manner such as that described in U.S. Patent Specification 3,952,334, and coating is carried out until a thickness of about 0.3 μm of carbon is deposited on the surfaces of the individual fibres at the exterior of the suture 20, with decreasing thickness towards the interior of the suture. The carbon coating is smooth and uniform, and has a density of about 1.8 g/cm$^3$, a BAF of about 1.2, and a tensile strain at fracture which is greater than 5 per cent.

Like the fibres of the embodiment 10 of Figure 1, the substrate fibres of the suture 20 or other sutures such as a tendon or heart valve suture are individually coated with the carbon coating and are not substantially bonded together. The individual fibres are thus free to bend and glide over each other in the flexure of the suture. The sutures have excellent compatibility with blood and tissue at the suture site.

It will be appreciated that in accordance with the present invention, multifibre sutures have been provided which are particularly adapted for prolonged or permanent implantation in the living body, which are biologically inert, which have substantial tensile strength, and which are capable of substantial flexible motion in service.

1

## C L A I M S

1.  A multifibre suture for prolonged or permanent implantation in a living body, comprising

a fibre substrate array of a plurality of organopolymeric fibres having a tensile modulus of elasticity of at least $13.8 \times 10^6$ kPa, and a fibre diameter of less than 25 µm, and

a dense, isotropic carbon coating on the fibres having a BAF (Bacon Anisotropy Factor) of not more than 1.3, a thickness of less than 0.7 µm, and a tensile fracture strain of at least 5 percent.

2.  A suture in accordance with claim 1, wherein the substrate array is a helically twisted array of said fibres.

3.  A suture in accordance with claim 1 or 2, wherein the substrate fibre is polyethylene terephthalate.

4.  A suture in accordance with claim 1, 2 or 3, wherein the array is a braided array of said fibres.

5.  A suture in accordance with any one of claims 1 to 4, wherein the suture comprises at least seven of said fibres.

6.  A suture in accordance with any one of claims 1 to 5, wherein the fibres have a tensile strength of at least $69 \times 10^3$ kPa.

0002090

# FIG.1

10

# FIG.2

10
12

# FIG.3

24
20
22
26

## DOCUMENTS CONSIDERED TO BE RELEVANT

| Category | Citation of document with indication, where appropriate, of relevant passages | Relevant to claim |
|---|---|---|
| DA | US - A - 3 526 005 (J.C. BOKROS)<br>* Column 8, lines 5-26 * | 1-6 |
| A | US - A - 3 557 795 (W. HIRSCH)<br>* Column 5, claim 1 * | 1-6 |
| A | US - A - 3 390 681 (L.D. KURTZ)<br>* Column 5, claim 1 * | 1-6 |
| A | US - A - 3 187 752 (A. GLICK)<br>* Column 8, claim 1 * | 1-6 |

**CLASSIFICATION OF THE APPLICATION (Int. Cl.²)**

A 61 L 17/00

**TECHNICAL FIELDS SEARCHED (Int.Cl.²)**

A 61 L 17/00

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 26-02-1979 | PELTRE |

EPO Form 1503.1 06.78